# FASCICULE DE BREVET EUROPEEN

(11) **EP 0 725 140 B1**
(45) Date de publication et mention de la délivrance du brevet: **17.09.2003**
(21) Numéro de dépôt: 96400201.8
(22) Date de dépôt: 29.01.1996
(51) Int. Cl.: C12N 15/67, C07K 1/14, C12P 21/02, C07K 14/54, C07K 14/61

(54) **Procédé d'extraction de protéines périplasmiques de microorganismes procaryotes en présence d'arginine**
Verfahren zur Extraktion von periplasmatischen Proteinen aus prokaryotischen Mikroorganismen in der Anwesenheit von Arginin
Process of extraction of periplasmic proteins from prokaryotic microorganisms in the presence of arginine

(30) Priorité: 31.01.1995 FR 9501083
(43) Date de publication de la demande: 07.08.1996
(73) Titulaire: SANOFI-SYNTHELABO, 75013 Paris (FR)
(72) Inventeur: Legoux, Richard, F-31460 Le Faget (FR); Maldonado, Paul, F-69360 St. Symphorien d'Ozon (FR); Salome, Marc, F-31320 Castanet Tolosan (FR)
(74) Mandataire: Gillard, Marie-Louise

(56) Documents cités:
- EP-A- 0 217 379
- EP-A- 0 360 641
- EP-A- 0 393 725
- WO-A-88/10307
- WO-A-92/22665
- PATENT ABSTRACTS OF JAPAN vol. 11, no. 232 (C-437), 29 Juillet 1987 & JP-A-62 044198 (HANAKA KEIZO), 26 Février 1987,
- PATENT ABSTRACTS OF JAPAN vol. 11, no. 246 (C-439), 11 Août 1987 & JP-A-62 051983 (YOSHIHIDE HAGIWARA), 6 Mars 1987,
- DISS. ABSTR. INT. B, vol. 37, no. 5, 1976, pages 200-201, XP002000663 BROWN ET AL.: "Purification by bioaffinity chromatography of two arginine-specific periplasmic binding proteins"

## Description

La présente invention concerne l'extraction des protéines recombinées produites par des microorganismes procaryotes, en particulier par E. coli.

On fait de plus en plus appel aux techniques de génie génétique pour la production de protéines d'intérêt, telles que par exemple l'insuline, les interleukines, l'hormone de croissance, etc.

Généralement le microorganisme est transformé par un vecteur d'expression comportant un gène codant pour la protéine d'intérêt et des moyens nécessaires à son expression tels que les signaux de régulation. Ensuite le microorganisme est cultivé sur un milieu de culture approprié et suivant des paramètres de culture appropriés et lorsqu'un nombre suffisant de cellules de microorganismes a été atteint, l'addition d'un inducteur déclenche la phase dite d'expression au cours de laquelle la protéine recherchée est produite à haut niveau et s'accumule. En fin de culture les cellules en suspension sont séparées du milieu de culture, par exemple par centrifugation ou microfiltration, puis sont soumises à un procédé d'extraction qui commence fréquemment par une opération de cassage des parois des microorganismes.

L'expression d'un gène codant pour une protéine d'intérêt dans un microorganisme procaryote peut être cytoplasmique, périplasmique ou sécrétoire selon la nature des moyens d'expression mis en oeuvre avec ledit gène (promoteur, terminateur, site de fixation des ribosomes, peptide-signal, etc.).

L'expression cytoplasmique permet d'obtenir des quantités importantes de protéines. Toutefois, préalablement à l'extraction de la protéine d'intérêt, il est nécessaire pour les protéines comprenant un ou plusieurs ponts disulfures de procéder à une étape de dénaturation/renaturation, ce qui représente une étape particulièrement lourde et délicate lors de la production à l'échelle industrielle. L'étape de dénaturation/renaturation est réalisée selon des moyens classiques bien connus de l'homme du métier, en utilisant un agent de dénaturation en présence d'un agent réducteur, puis des conditions de renaturation comprenant notamment un contrôle du redox de la solution. Parmi les agents dénaturants utilisés il faut surtout citer le chlorhydrate de guanidine qui a été proposé dans un procédé d'obtention de l'interleukine-2 humaine. A cet effet, on peut se référer par exemple au document EP-A2-0 145 390.

Les systèmes d'expression sécrétoires dans lesquels la protéine d'intérêt se retrouve dans le milieu de culture de manière active sont peu ou pas utilisés chez les bactéries Gram négatives en raison de leur faible productivité. Il faut remarquer ici que le milieu d'une culture bactérienne à forte densité dans un bioréacteur n'est pas un lieu de séjour idéal pour des protéines recombinées sensibles en raison par exemple des risques de dénaturation interfaciale.

L'expression périplasmique permet d'obtenir directement des protéines recombinées en principe correctement repliées dans un espace protégé de l'environnement et de ce fait représente un choix judicieux pour l'obtention des protéines, notamment des protéines non glycosylées. Dans ce cas, il n'est donc pas nécessaire de soumettre les protéines à une étape de dénaturation-renaturation.

Les méthodes de cassage des cellules généralement utilisées dans ce domaine sont par exemple la lyse des cellules par sonication ou par pression mécanique (French Pressure Cell, moulin à billes), la lyse chimique ou la lyse enzymatique, le choc osmotique et le traitement à l'aide d'agents chaotropiques ou de détergents. Ces méthodes cassent la plupart des membranes cellulaires dont les membranes plasmiques et les membranes du réticulum endoplasmique pour former une suspension homogène de débris cellulaires. La nature du culot de débris cellulaires pouvant être récolté en général après centrifugation (noyaux, cytosquelette, mitochondries, lysosomes, ribosomes, macromolécules, etc.) est dépendante en particulier de la durée et de la vitesse de centrifugation (10 minutes à 1000 g jusqu'à 3 heures à 150 000 g).

Les difficultés rencontrées au cours des opérations d'extraction varient selon le type d'expression et les méthodes d'extraction utilisées et sont notamment :
- la perte en rendement de la protéine recombinée
- la perte d'activité biologique de la protéine recombinée
- la dégradation protéolytique de la protéine recombinée
- la toxicité des agents d'extraction et le contrôle obligatoire de leur élimination
- la difficulté de mise en oeuvre industrielle
- le mélange des protéines périplasmiques avec les protéines cytoplasmiques.

De plus, lorsque les protéines d'intérêt produites sont hydrophobes ou chargées, elles peuvent s'associer à des composants cellulaires qui sont eux-mêmes hydrophobes ou chargés, ce qui rend l'extraction particulièrement difficile.

La production industrielle de protéines recombinées d'intérêt par génie génétique représente un enjeu considérable, mais nécessite la mise au point de méthodes d'extraction qui évitent ou minimisent les inconvénients ci-dessus.

En effet, il est non seulement important de produire des quantités importantes de protéine d'intérêt, mais encore faut-il que ces protéines ne soient pas contaminées par les agents d'extraction et conservent toute leur activité biologique.

Différents procédés ont été proposés à cet effet, en particulier pour l'extraction-séparation de l'interleukine-2.

Le document EP-A2-0 145 390 décrit un procédé pour l'obtention d'interleukine-2 (IL-2) non glycosylée humaine ayant une activité spécifique supérieure à 104 U/mg qui met en oeuvre une étape de séparation par chromatographie sur colonne pour extraire l'IL-2. Ce procédé fait également intervenir une étape de dénaturation à l'aide du chlorhydrate de guanidine.

Le document EP-A2-0 147 819 propose un procédé d'obtention d'interleukine-2 recombinante homogène et pure. Ce procédé consiste à cultiver un microorganisme transformé à l'aide d'un vecteur d'expression contenant le gène codant pour l'interleukine-2, à provoquer la lyse des cellules, à récupérer les débris cellulaires, à extraire l'IL-2 par lavage des débris cellulaires avec une solution de lavage appropriée, puis à purifier par chromatographie la solution de lavage. Les solutions de lavage utilisées peuvent contenir un sel, tel que le chlorure de sodium ou le chlorhydrate de guanidine, ou un détergent, tel que par exemple le produit connu sous la dénomination commerciale "Triton X®-100".

Selon une variante préférée, on préconise la mise en oeuvre successive de trois solutions de lavage à savoir : une solution de lavage contenant du chlorure de sodium, une solution de lavage contenant un détergent et une solution de lavage contenant du chlorhydrate de guanidine.

Le document EP-A1-0 337 243 décrit un procédé pour purifier l'interleukine-2 humaine qui utilise un système de deux colonnes de chromatographie liquide en phase inverse. Avant l'étape de purification par chromatographie, la fraction insoluble du lysat de cellules bactériennes est extraite avec une solution contenant du chlorhydrate de guanidine pour obtenir un extrait bactérien, lequel est ensuite dilué à l'aide d'un tampon exempt de chlorhydrate de guanidine puis chromatographié, l'élution étant réalisée avec un gradient d'acétonitrile.

On a maintenant trouvé de façon surprenante que l'extraction d'une protéine d'intérêt produite par un microorganisme procaryote, transformé par un vecteur d'expression comportant un gène codant pour la protéine d'intérêt et des moyens pour son expression tels que les signaux de régulation nécessaires à son expression périplasmique, peut être réalisée par mise en suspension du culot de cellules ou de débris cellulaires du microorganisme, provenant de la culture dudit microorganisme, dans une solution tampon, ladite solution contenant avantageusement de l'arginine, arginine pouvant être sous la forme L ou/et D.

Selon un premier aspect, l'invention a pour objet l'utilisation de l'arginine comme agent d'extraction des protéines périplasmiques.

Selon un autre aspect, la présente invention a pour objet un procédé d'extraction d'une protéine périplasmique d'intérêt qui consiste :
1/ à mettre en suspension le culot de cellules provenant de la culture d'un microorganisme transformé par un vecteur d'expression comportant un gène codant pour ladite protéine et tous les signaux de régulation nécessaires à son expression périplasmique dans une solution tampon contenant de l'arginine et, après un temps de contact dans des conditions de pH, de température, de concentration bactérienne etc...; adéquates,
2/ à récupérer la protéine d'intérêt dans le surnageant de la suspension bactérienne ainsi obtenue.

Une variante dudit procédé d'extraction d'une protéine d'intérêt périplasmique consiste à mettre en suspension le culot de débris cellulaires, obtenu après lyse des cellules provenant de la culture, dans la solution tampon contenant de l'arginine.

L'extraction des protéines périplasmiques est particulièrement efficace quand le tampon d'extraction est constitue par une solution aqueuse contenant de l'arginine à une concentration au moins égale à 0,4 M d'arginine dans la limite de solubilité de l'arginine à température ambiante dans l'eau (voisine de 0,8 M dans l'eau pure et au delà en présence de sels), et que son pH est légèrement alcalin, de préférence égal à 8.

L'arginine est un acide α-aminé naturel qui a été proposé comme agent auxiliaire de dénaturation/renaturation/substitution de deux chaînes de l'Abbokinase® (activateur urinaire du plasminogène) dans lesquelles un peptide natif est remplacé partiellement par un peptide synthétique au cours de cette opération. A cet effet, on peut se référer à l'article de GA. Homandberg et T. Wai dans Biochimica et Biophysica Acta, 1990,1038, 209-215.

Dans le procédé de l'invention ou sa variante, on ne procéde pas à la dénaturation/renaturation de la protéine et l'arginine intervient uniquement au niveau de l'extraction d'une protéine à partir d'un culot de cellules ou de débris cellulaires de microorganismes.

L'arginine procure des effets remarquables sur l'extraction de la protéine tant au niveau du rendement que de l'activité biologique de la protéine. On a en effet trouvé que, par exemple, la forme mature de l'IL-13 est récupérée avec le procédé de l'invention avec des rendements supérieurs à 95 % tout en conservant l'activité biologique ce celle-ci. Il faut noter que ces essais d'extraction par choc osmotique sur le même système d'expression n'aboutissent pas à des rendements comparables.

Des essais comparatifs ont montré que la guanidine.HCI utilisée dans les mêmes conditions permet aussi de récupérer la protéine IL-13 avec un rendement supérieur à 95% ; en revanche l'activité biologique de la protéine ainsi récupérée est altérée davantage que par la méthode à l'arginine.

Sans vouloir se limiter à une quelconque théorie, on pense que l'arginine agit comme un agent chaotropique doux et biologique par opposition aux agents chaotropiques puissants et dénaturants aux concentrations élevées nécessaires, égales ou supérieures à 5 M, pour assurer l'extraction, tel que le chlorhydrate de guanidine.

Le procédé de l'invention ou sa variante peuvent être mis en oeuvre à la suite d'un procédé quelconque de culture d'un microorganisme transformé avec un vecteur d'expression contenant un gène codant pour la protéine d'intérêt, et des moyens pour une expression périplasmique de ladite protéine tels que tous les signaux de régulation nécessaires.

Il est évident pour l'homme de l'art que la méthode est applicable aux bactéries proches de E.coli, c'est à dire aux bactéries dites Gram négatives anaérobies facultatives constituant le groupe des Entérobactéries. Dans cette famille des Entérobactéries nous trouvons en particulier les espèces: Escherichia, Salmonella, Erwinia mais aussi Shigella, Klebsiella, Serratia, Proteus et Enterobacter.

Compte tenu du caractère chaotropique de l'arginine il apparaît également que l'arginine peut selon les cas se substituer avantageusement à d'autres agents chaotropiques. Sans pouvoir exemplifier sur toutes les familles de bactéries en raison de la diversité des systèmes vivants en cause, l'homme de l'art saura appliquer et adapter la méthode d'extraction à l'arginine à son cas particulier.

De tels procédés de culture sont bien connus de l'homme du métier. Des procédés décrivant la culture de bactéries Gram négatives en fermenteur sont décrits par exemple dans le brevet EP-360641 et EP-356335 relatant l'obtention et l'utilisation des souches E.coli dites SEBR 1250 et TP 2339.

Lorsque le nombre de cellules désiré a été atteint, la culture est soumise à une centrifugation (en général) ou une microfiltration et le culot de biomasse obtenu est mis en contact avec une solution tampon contenant de l'arginine selon le procédé de l'invention.

En règle générale on opère à une température comprise entre la température ambiante d'environ 25°C et 2°C, de préférence à 4°C.

Le temps de contact du culot de cellules avec la solution tampon contenant de l'arginine doit être suffisant pour permettre le passage de la protéine d'intérêt dans la solution tampon.

En général, lorsqu'on opère à 4°C, le temps de contact est avantageusement de 1 heure environ.

L'extraction, c'est à dire le passage de la protéine périplasmique dans le milieu, se poursuit au cours de la mise en contact de la biomasse et du tampon d'extraction à l'arginine. Le temps de contact assurant une extraction complète ou n'évoluant plus est compris entre 30 minutes et 16 heures. Des essais montrent que des rendements d'extraction satisfaisants peuvent être obtenus en l'espace de quelques heures à la température de 4°C. Il a été noté également qu'une agitation douce de la biomasse dans son tampon d'extraction de manière à éviter la sédimentation du culot de microorganismes donne des résultats supérieurs, c'est à dire des taux d'extraction plus élevés en fonction du temps.

Le procédé d'extraction selon l'invention convient pour extraire aussi bien les protéines hydrophobes, telles que par exemple les interleukines, en particulier l'IL-13 décrite dans le document EP-A1-0 506 574, que les protéines hydrophiles, telles que par exemple l'hormone de croissance (hGH). Le procédé de l'invention simplifie l'obtention de l'hGH qui nécessite normalement pour son extraction la mise en oeuvre d'un choc osmotique.

Pour réaliser l'extraction de la protéine d'intérêt directement sur la suspension du culot de cellules, une solution tampon contenant de l'arginine à une concentration comprise entre 0,4 M et 0,8 M sera préférée.

Lorsque l'on veut réaliser l'extraction de la protéine périplasmique d'intérêt sur le culot de débris cellulaires selon la variante du procédé de l'invention, on procède de la même manière que selon le procédé de l'invention jusqu'à l'étape d'obtention du culot de cellules obtenu après centrifugation ou microfiltration, puis on effectue le cassage des cellules suivant des méthodes bien connues de l'homme du métier. Des méthodes de cassage de cellules sont décrites par exemple dans C.T. Chôma and H. Yamazaki, Can. J. Microbiol., 1981, 27, 547-550 ; LO. Ingram, Journal of Bacteriology, 1981, *146,1,* 331-336 ; N.G. Nossal and LA. Heppel, Journal of Biological Chemistry, 1966, 241, 13, 3065-3072 ; R. Bennett, D.R. Taylor and A. Hurst, Biochem. Biophys. Acta, *18 (3),* 512-521 (1966), et dans l'ouvrage collectif Fermentation and enzyme technology, Chap. 12, 239-309, J. Wiley and Sons Editeurs (1979).

Le culot de débris cellulaires récolté en règle générale après centrifugation est remis en suspension, puis mis en contact avec une solution tampon contenant de l'arginine. Le temps de contact de la suspension de débris cellulaires avec la solution tampon contenant de l'arginine doit être suffisant pour permettre le passage de la protéine d'intérêt dans la solution tampon. En général, pour une température de 4°C, le temps de contact assurant une extraction pratiquement complète est de 48 heures. De la même manière, il a été noté qu'une agitation douce de la biomasse dans son tampon d'extraction, évitant ainsi la sédimentation des débris cellulaires, donne des taux d'extraction plus élevés en fonction du temps.

Cette variante du procédé d'extraction selon l'invention convient pour extraire en particulier les protéines périplasmiques d'intérêt qui sont fortement associées aux membranes cellulaires telles que par exemple les interleukines.

Il est bien connu de l'homme du métier que le tampon d'extraction contenant l'arginine selon l'invention, pourra également contenir un détergent auxiliaire qui aura pour effet d'améliorer le rendement et/ou la vitesse d'extraction de la protéine d'intérêt. Parmi les détergents auxiliaires que l'on pourra utiliser, l'homme du métier saura choisir parmi ceux qui permettent de préserver les avantages de l'utilisation de l'arginine comme agent d'extraction, en particulier la conservation de l'activité biologique de la protéine d'intérêt. Parmi ces détergents auxiliaires doux on peut citer par exemple les alkyl-glycosides comme les alkyl-maltosides, les nonyl-α ou β-D-glucopyranosides, les octyls-α ou β-D-glucopyranosides ou les alkyl-carbamoyl-méthyl-α ou β-D-glucopyranosides comme par exemple l'Hecameg®, dont la très faible toxicité laisse envisager la possibilité de pouvoir le retrouver à l'état de trace comme agent de formulation dans le produit final.

Pour réaliser l'extraction de la protéine d'intérêt à partir de la suspension du culot de débris cellulaire, on préférera utiliser une solution tampon contenant de l'arginine à une concentration comprise entre 0,4 M et 2,5 M, une concentration de 2,5 M d'arginine pouvant être obtenue en particulier en présence de sels.

L'invention va être maintenant décrite plus en détail à l'aide des EXEMPLES ci-après donnés uniquement à titre illustratif.

### EXEMPLE 1

Extraction de I'IL-13 périplasmique de E. coli en présence d'arginine sur culot de cellules

### 1/ Culture en fiole :

Dans cet exemple, on a utilisé la souche E. coli RB 791 (Roger Brent, PNAS 78 (1981) p. 4204-4208) transformée par le plasmide p922 obtenu selon des procédés analogues à ceux définis dans les brevets EP 360 641 et 356 335 et dont la séquence d'ADN est la séquence SEQ ID No.1.

Les différentes séquences qui constituent ce plasmide p922 sont indiquées ci-après.

### SEQUENCE DU PROMOTEUR

Les hexanucléotides TTGCTT et TATAAT caractéristiques des promoteurs chez E.coli sont indiqués en caractère gras

### SEQUENCE DE LA REGION 5 PRIME NON TRADUITE DU MESSAGER

Le site de fixation au ribosome est indiquée en caractère gras. La sequence CAT située à l'extrémité 3 prime de cette séquence est une partie de l'héxanucléotide reconnu par l'enzyme de restriction Ndel

### SEQUENCE CODANT POUR LE PRECURSEUR DE L'IL-13

La séquence en italique correspond à la séquence de l'IL-13 mature. La séquence qui n'est pas en caractère gras est une séquence de liaison liant la fin de la séquence codant pour l'IL-13 à l'héxanucléotide reconnu par l'enzyme de restriction BamHl

### SEQUENCE DE TERMINAISON

### TERMINATEUR DU GENE 10 DU PHAGE T7

### TERMINATEUR DU PHAGE fd

### GENE CODANT POUR LE REPRESSEUR DE L'OPTION LACTOSE

### SEQUENCE DE pBR 327

(le plasmide pBR 327 est décrit dans Gene, 9, 287-305 (1980))

Cette souche E. coli RB 791/p922 a été mise en préculture une nuit à 30°C sous agitation à 200 tr/min sur du milieu L (Luria broth décrit dans Molecular Cloning, A Laboratory Manual Sambrook, Fritsch, Maniatis ; Cold Spring Harbor Laboratory Press., 2eme édition 1989) avec 100 mg/l d'ampicilline. A partir de cette préculture une nouvelle fiole de milieu L a été inoculée de telle sorte que la D.O. (D.O. = Densité Optique à 600 nm, D.O. = 1 correspond à 400-450 mg biomasse/litre) initiale soit de 0,6. Après une heure d'attente la culture a été induite avec 1 mM d'IPTG (isopropyl-β-D-1-thiogalactopyranoside) et poursuivie pendant 3 heures. Les échantillons de la suspension bactérienne ont été centrifugés et les culots bactériens ainsi récupérés ont été mis en suspension dans les tampons d'extraction ci-après, de telle sorte que la D.O finale soit de 10, ce qui équivaut à 4,5 g biomasse/ litre, au moment de l'extraction.

Les tampons d'extraction utilisés dans cet exemple sont les suivants:
A: Arginine 0,8 M pH 8,0 corrigé par HCI dans eau Milli-Q® (Millipore)
B: Guanidine.HCI 5 M dans eau Milli-Q® sans correction de pH.

L'extraction a été effectuée en 1 heure à 4°C sous agitation magnétique légère.

Pour mesurer l'efficacité d'extraction, des échantillons équivalents à à ml de suspension de culture à D.O 0,2 ont été prélevés et les culots bactériens correspondants obtenus par centrifugation à 5 000 g pendant 10 min ont été déposés sur gel de polyacrylamide à 16,5 % après dénaturation au SDS. Les suspensions bactériennes ont été également centrifugées et leurs surnageants ont été dessalés par ultrafiltration (dispositif de filtration Ultrafree-MC de seuil de coupure 5 000 Da Millipore) avant d'être déposés sur gel. Le gel lui même a été révélé par Western blot en utilisant un anticorps anti-IL-13 CHO et quantifié au Phosphorlmage® ( Molecular Dynamics). L'anticorps anti-IL-13 CHO (Chinese Hamster Ovary) utilisé dans cet exemple a été obtenu par immunisation de lapins.

On a constaté dans cet exemple que l'extraction en présence de guanidine.HCl ou bien en présence d'arginine est quasiment complète pour la forme mature avec des rendements d'extraction supérieurs dans les deux cas à 99%. On a également noté que dans le surnageant extrait en présence d'arginine la forme précurseur de l'IL-13 n'apparaît pas à la différence de l'extrait obtenu en présence de guanidine.HCl

### 2/ Culture en fermenteur :

La souche E.coli RB 791/ p922 a été lancée sur milieu L avec 100 mg/l d'ampicilline et incubée à 30°C sous agitation pour constituer une préculture. Un volume de 100 ml de cette préculture a servi d'inoculum à un fermenteur de 2,5 litres de volume total de marque MBR. La culture a été effectuée dans un volume de 1,2 litre sur un milieu dont la composition est donnée ci-après et dans les conditions définies ci-après.

### Milieu pour fermenteur - souche E.coli RB 791/ p922

La formule est donnée pour 1 litre final, le volume de l'inoculum est à retrancher.

### 1. Dissoudre dans 700 ml d'eau Milli-Q®:

| Composant | Masse/litre |
|---|---|
| EDTA | 1 g |
| FeSO₄, 7H₂O | 45 mg |
| MgSO₄, 7H₂O | 1,5 g |
| K₂SO₄ | 0,75 g |
| CaCl₂, 2H₂O | 32 mg |
| NaCl | 1,45 g |
| KCI | 5 g |
| HY-SOY® | 75 g |
| L-méthionine | 1,4 g |
| Tryptophane | 1 g |
| Oligoéléments∗ | 2 ml |
| Extrait de levure | 10 g |

Compléter à 800 ml avec de l'eau Milli-Q®, autoclaver 30 min. à 120°C.

### 2. Filtrer sur 0.2 um dans 100 ml d'eau Milli-Q®

| | |
|---|---|
| Glycérol | 15 g |
| K₂HPO₄ | 7,1 g |

La concentration en glycérol sera maintenue entre 10 et 15 g/l au cours de la culture.

### 3. Au moment de l'induction ajouter

| | |
|---|---|
| IPTG | 1 g |
| Acid 6-aminocaproïque | 0,65 g |
| HY-SOY® | 40 g |
| L-cystéine | 0,3 g |

Le volume de cet ajout n'est pas inclus dans les autres calculs.

### * Solutions d'oliqoéléments

Elle est utilisée à 1 ml/litre

Pour 1 litre d'eau Milli-Q® final, dissoudre dans 800 ml :

| | masse/l |
|---|---|
| ^{H}3^{BO}3 | 3 mg |
| NaMoO₄2 H₂O | 4,8 mg |
| MnSO4, H₂O | 59 mg |
| GoCl₂, 6 H₂O | 23,8 mg |
| CuSO₄, 5 H₂O | 8,7 mg |
| ZnS0₄, 7H₂0 | 13 mg |
| AICI₃, 6 HₛO | 60 mg |
| ^{KCr}(^{S0}4)2, ^{12 H}20 | 6 mg |
| Kl (extemporanément) | 60 mg |
| NiSO₄, 6 H₂O | 2,6 mg |

Ajouter 100 ml d'HCI concentré. Compléter à 1000 ml avec eau Milli-Q®.

Une fois la D.O. de 58 atteinte l'expression de l'IL-13 est déclenchée par l'addition d'IPTG à 1 g/l et poursuivie pendant 5 heures.

Les paramètres de culture en fermenteur étaient les suivants:
pH = 7,4
T = 30°C
pO₂ = 40 mm Hg régulée par l'agitation, avec un débit d'air compris entre 1 et 3 litres/min.

Les méthodes d'extraction et de mesure de l'activité biologique appliquées sont les mêmes que celles décrites au paragraphe 1 ci-dessus.

On a constaté que l'extraction - sur un culot bactérien obtenu en fermenteur et non plus en fiole - en présence de guanidine.HCI ou bien en présence d'arginine, est quasiment complète pour la forme mature de l'IL-13 avec des rendements d'extraction supérieurs dans les deux cas à 97%.

### EXEMPLE 2

Activité biologique de l'IL-13 ainsi extraite

Les extraits obtenus en présence de guanidine.HCI ou d'arginine dans l'exemple 1 ont été dessalés par ultrafiltration comme décrit ci-dessus. Ils ont été mis après dilution en série en présence d'un sous-clone IL-13-dépendant de la lignée cellulaire B9. L'activité IL-13 des échantillons dilués induit la croissance des cellules B9 et la concentration de demi-prolifération a été déterminée. La croissance cellulaire a été arrêtée après 3 jours de contact par addition de MTT [bromure de 3-(4,5-diméthylthiazol-2-yl-2,5-diphényltétrazolium)] et mesurée au spectrophotomètre par absorption de la coloration bleue provoquée à 565 nm. L'activité biologique IL-13 a été exprimée en ng/ml par rapport à un standard IL-13 lui même calibré sur le standard international candidate obtenu à partir de culture IL-13 CHO obtenue par immunisation de lapins selon N. Vita, Archives of Biochemistry and Biophysics, 1983, *225,* 2, 436-445.

Les résultats obtenus figurent dans le TABLEAU II ci-après.

**TABLEAU II**

| Essai sur lignée cellulaire B9 | IL-13 en ng/ml | Activité biologique en ng/ml | Activité biologique spécifique |
|---|---|---|---|
| Témoin | 500 | 500 | 100 |
| Arginine | 3 200 | 1376 | 43 |
| Guanidine | 4 300 | 1 098 | 25 |

Les résultats ci-dessus montrent que l'activité biologique spécifique de l'extrait à l'arginine est, avant toute autre opération de purification ultérieure, supérieure à celle de l'extrait au chlorhydrate de guanidine.

### EXEMPLE 3

Extraction de l'hGH périplasmique de E.coli en présence d'arginine sur culot de cellules.

La souche SEBR 1250 (EP-360641 et EP-356335) a été mise en préculture une nuit à 37°C sous agitation à 200 tr/min sur du milieu L (Luria broth) avec 100 mg/l d'ampicilline. A partir de cette préculture, une nouvelle fiole de milieu L a été inoculée de telle sorte que la D.O. initiale soit de 0,2. Après une heure d'attente la culture a été induite avec 1 mM d'IPTG et poursuivie pendant 3 heures. Les échantillons de la suspension bactérienne ont été centrifugés et les culots bactériens ainsi récupérés ont été mis en suspension dans les tampons d'extraction de telle sorte que la D.O finale soit de 10, ce qui équivaut à -4,5 g biomasse/ litre, au moment de l'extraction.

Les conditions d'extraction ont été les suivantes :

| Agent chaotropique | pH | T | durée |
|---|---|---|---|
| Arginine 0,8 M | 8,0 | 22°C | 20 heures |
| Arginine 0,8 M | 8,0 | 4°C | 20 heures |

Pour mesurer l'efficacité d'extraction, des échantillons équivalents à 1 ml de suspension de culture à D.O 0,2 ont été prélevés et les culots bactériens correspondants obtenus par centrifugation à 5 000 g pendant 10 min ont été déposés sur gel de polyacrylamide à 16,5 % après dénaturation au SDS. Les suspensions bactériennes ont été également centrifugées et leurs surnageants déposés sur gel. Le gel lui même a été révélé par Western blot en utilisant un anticorps anti-hGH et quantifié au Phosphorlmager® ( Molecular Dynamics). Les anticorps anti-hGH utilisés ont été obtenus par immunisation de lapins.

L'analyse des bandes obtenues par Phosphorlmage® permet de conclure que l'extraction de l'hGH périplasmique humaine produite chez E.coli en présence d'arginine est efficace. Dans cet exemple un rendement d'extraction d'au moins 60 % peut être atteint en présence d'arginine 0,8 M, pH 8,0, T 22°C et 20 heures d'attente, et un rendement d'extraction supérieur à 80% peut être atteint en présence d'arginine 0,8 M, pH 8,0, T 4°C et 20 heures d'attente.

L'hGH étant une protéine hydrophile, on peut en conclure que des protéines recombinantes de nature très différentes accumulées dans le périplasme de E.coli peuvent être extraites simplement en présence d'arginine.

### EXEMPLE 4

Extraction de l'IL-13 périplasmique de E. coli sur débris cellulaires en présence d'arginine.

### 1/ Culture en fermenteur

Dans cet exemple, on a utilisé la souche E. coli TP2339 (EP 360641 et EP356335) transformée par le plasmide p922 obtenu selon des procédés analogues à ceux définis dans l'EXEMPLE 1.

La souche E.coli TP2339/p922 a été lancée sur milieu L avec 100 mg/l d'ampicilline et incubée à 30°C sous agitation pour constituer une préculture. Un volume de 100 ml de cette préculture a servi d'inoculum à un fermenteur de 2,5 litres de volume total de marque MBR®. La culture a été effectuée dans un volume de 1,2 litre dans un milieu et des conditions définis ci-après.

### Milieu pour fermenteur - souche E.coll TP2339/p922

Calculé pour un volume final de 1,2 litre, le milieu de culture est constitué par l'addition de un litre de phase autoclavée et 0,1 litre de phase filtrée dont les compositions sont décrites ci-dessous, et de 0,1 litre de préculture, définie ci-dessus.

### 1/ Phase autoclavée (1000 ml) :

Dissoudre dans 900 ml d'eau Milli-Q®

| | Masse/litre |
|---|---|
| Tricine | 360 mg |
| FeSO₄, 7H₂O | 280 mg |
| CaCl₂, 2H₂O | 6,7 mg |
| MgCl₂, 6H₂O | 1,27 g |
| K₂SO₄ | 8,71 g |
| NaCl | 500 mg |
| KCl | 5 g |
| Hy-Case (SF)® | 25 g |
| Extrait de levure | 18 g |
| Oligoéléments∗ | 1 ml |
| L-arginine | 1,5 g |

Ajuster le pH à 7,4 avec une solution de KOH puis compléter à 1000 ml avec de l'eau Milli-Q®. Autoclaver 30 minutes à 120°C.

### 2/ Phase filtrée (100 ml)

Filtrer stérilement sur membrane 0,2 µm :

| Glucose | 20 g |
|---|---|
| Glycérol | 50 g |
| K₂HPO₄ | 5 g |

La concentration en glucose sera maintenue au cours de la culture à une concentration comprise entre 5 et 15 g/l.

Une fois la D.O. de 40 atteinte (environ 16 g de matière sèche/litre), l'expression de l'IL-13 est déclenchée par l'addition d'IPTG à 1 g/l et poursuivie pendant 5 heures. Les paramètres de culture étaient les suivants:
pH = 7,4 régulé par KOH et HCI 3N
T=37°C
P0₂ = 50 mbars régulé par l'agitation avec un débit d'air compris entre 1 et 3 litres/min.

### 2) Récupération et broyage des corps bactériens

Un litre de suspension de culture est centrifugée 20 minutes à ~ 6400 g. Le culot est repris dans le même volume de tampon Tris 10 mM, EDTA 1 mM, pepstatin 1 mg/l, pH 8 sous agitation mécanique à hélice.

Le broyage est accompli dans une presse Manton-Gaulin à une pression de 700 bars au cours de deux passages. Le broyat tel que peut être conservé à-80°C dans cet exemple.

### 3) Extraction

Après décongélation, 5 ml du broyat de D.O. égale à 75 (30 g de matière sèche/litre) sont prélevés puis centrifugés 50 minutes à 23 300 g.

Le culot ainsi obtenu est repris dans le tiers du volume initial avec du tampon Tris 0,1 mM pH 7,0, puis complété au volume initial avec une solution contenant de l'arginine de telle sorte que la concentration finale en arginine soit de 2,5 M et le pH de 8,0.

Pour cet exemple, un détergent auxiliaire (Hecameg® à 20 g/l de concentration finale) a été associé à l'arginine.

La suspension de débris cellulaires ainsi constituée est placée à 4°C sur un agitateur rotatif à 300 tr/minute pendant 2 jours.

La suspension est alors centrifugée une dernière fois 50 minutes à 23 300 g, le surnageant constituant l'extrait attendu.

### 4) Analyses biochimique et d'activité biologique

a) Le dosage des protéines totales a été effectué par la méthode "Protein Assay" de Biorad®.
b) La méthode de dosage de l'IL-13 recombinée est celle utilisée dans l'exemple 1.

### Rendement de l'IL-13 ainsi extraite.

Les résultats obtenus sont décrits dans le tableau suivant:

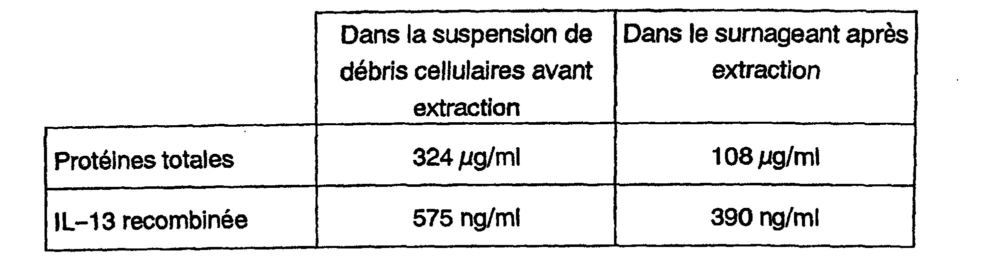

On a constaté dans cet exemple que l'extraction réalisée sur débris cellulaires en présence d'arginine à 2,5 M et d'un détergent auxiliaire permettait d'obtenir un rendement d'extraction d'environ 70 %.

L'intérêt du procédé d'extraction à l'arginine selon l'invention est de pouvoir utiliser les extraits de protéines tels que ou avec un traitement minimum dans des tests d'activité biologique.

Cette simplification de la méthode d'extraction apporte un avantage tant pour la production industrielle de protéines recombinées périplasmiques que pour le criblage par dosage de l'activité biologique à l'échelle du laboratoire portant par exemple sur des protéines mutées.

A la différence de la guanidine.HCI, fréquemment utilisée comme agent d'extraction, l'arginine n'est pas agressive vis à vis des matériaux employés dans l'industrie, notamment les aciers. D'autre part l'arginine est un agent non polluant qui ainsi ne nécessite pas de procédé de traitement coûteux des effluents.

## Revendications

1. Utilisation de l'arginine comme agent d'extraction de protéines recombinées périplasmiques.

2. Procédé d'extraction d'une protéine périplasmique d'intérêt qui consiste :
1/ à mettre en suspension le culot de cellules provenant de la culture d'un microorganisme procaryote transformé à l'aide d'un vecteur d'expression contenant un gène codant pour ladite protéine et des moyens pour son expression dans le périplasme de celui-ci dans une solution tampon contenant de l'arginine ;
2/ à récupérer la protéine d'intérêt dans le surnageant de la suspension bactérienne ainsi obtenue.

3. Procédé d'extraction d'une protéine périplasmique d'intérêt qui consiste :
1/ à mettre en suspension le culot de débris cellulaires de cellules provenant de la culture d'un microorganisme procaryote transformé à l'aide d'un vecteur d'expression contenant un gène codant pour ladite protéine et des moyens pour son expression dans le périplasme de celui-ci dans une solution tampon contenant de l'arginine;
2/ à récupérer la protéine d'intérêt dans le surnageant de la suspension bactérienne ainsi obtenue.

4. Procédé selon la revendication 2 ou 3, **caractérisé en ce que** la solution tampon contenant de l'arginine est une solution aqueuse alcaline, ayant une concentration en arginine au moins égale à 0,4 M.

## Patentansprüche

1. Verwendung von Arginin als Mittel zur Extraktion von rekombinanten periplasmatischen Proteinen.

2. Verfahren zur Extraktion eines interessierenden periplasmatischen Proteins, das darin besteht:
1) den Bodensatz von Zellen, die aus der Kultur eines prokaryontischen Mikrorganismus stammen, der mit einem Expressionsvektor transformiert wurde, der ein Gen, das für das Protein codiert, und Mittel für seine Expression im Periplasma aufweist, in einer Pufferlösung, die Arginin enthält, zu suspendieren; und
2) das interessierende Protein in dem Überstand der auf diese Weise erhaltenen Bakteriensuspension zu gewinnen.

3. Verfahren zur Extraktion eines interessierenden periplasmatischen Proteins, das darin besteht:
1) den Bodensatz von Zellresten von Zellen, die aus der Kultur eines prokaryontischen Mikrorganismus stammen, der mit einem Expressionsvektor transformiert wurde, der ein Gen, das für das Protein codiert, und Mittel für seine Expression im Periplasma aufweist, in einer Pufferlösung, die Arginin enthält, zu suspendieren; und
2) das interessierende Protein in dem Überstand der auf diese Weise erhaltenen Bakteriensuspension zu gewinnen.

4. Verfahren nach Anspruch 2 oder 3, **dadurch gekennzeichnet, dass** die Pufferlösung, die das Arginin enthält, eine alkalische wässrige Lösung mit einer Argininkonzentration von mindestens 0,4 M ist.

## Claims

1. Use of arginine as an agent for the extraction of recombinant periplasmic proteins.

2. A method for the extraction of a periplasmic protein of interest, which consists in:
1/ suspending the pellet of cells originating from the culture of a prokaryotic microorganism, transformed by means of an expression vector containing a gene coding for the said protein and means for its expression in the periplasm of the said microorganism, in a buffer solution containing arginine; and
2/ recovering the protein of interest in the supernatant of the bacterial suspension thereby obtained.

3. A method for the extraction of a periplasmic protein of interest, which consists in:
1/ suspending the pellet of cell debris from cells originating from the culture of a prokaryotic microorganism, transformed by means of an expression vector containing a gene coding for the said protein and means for its expression in the periplasm of the said microorganism, in a buffer solution containing arginine; and
2/ recovering the protein of interest in the supernatant of the bacterial suspension thereby obtained.

4. The method according to Claim 2 or 3, **characterized in that** the buffer solution containing arginine is an alkaline aqueous solution having an arginine concentra-tion equal to at least 0.4 M.


### LISTE DE SEQUENCES

(1) INFORMATIONS GENERALES:
   (i) DEPOSANT:
      (A) NOM: SANOFI
      (B) RUE: 32-34 rue Marbeuf
      (C) VILLE: PARIS
      (E) PAYS: FRANCE
      (F) CODE POSTAL: 75008
      (G) TELEPHONE: 53.77.41.31
      (H) TELECOPIE: 53.77.42.16
   (ii) TITRE DE L' INVENTION: Procédé d'extraction de protéines périplasmiques de micro-organismes procaryotes en présence d'arginine
   (iii) NOMBRE DE SEQUENCES: 1
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 4410 paires de bases
      (B) TYPE: nucléotide
      (C) NOMBRE DE BRINS: simple
      (D) CONFIGURATION: linéaire
   (ii) TYPE DE MOLECULE: ADN (génomique)
   (ix) CARACTERISTIQUE:
      (A) NOM/CLE: promoter
      (B) EMPLACEMENT:1. .282
   (ix) CARACTERISTIQUE:
      (A) NOM/CLE: 5'UTR
      (B) EMPLACEMENT:283..337
      (D) AUTRES INFORMATIONS:/note= "séquence de la région 5' non traduite du messager"
   (ix) CARACTERISTIQUE:
      (A) NOM/CLE: misc RNA
      (B) EMPLACEMENT:338..762
      (D) AUTRES INFORMATIONS:/note= "séquence codant pour le précurseur de l'IL-13 "
   (ix) CARACTERISTIQUE:
      (A) NOM/CLE: terminator
      (B) EMPLACEMENT:763..812
   (ix) CARACTERISTIQUE:
      (A) NOM/CLE: terminator
      (B) EMPLACEMENT:813..1012
      (D) AUTRES INFORMATIONS:/note= "terminateur du gène 10 du phage T7"
   (ix) CARACTERISTIQUE:
      (A) NOM/CLE: terminator
      (B) EMPLACEMENT:1013..1253
      (D) AUTRES INFORMATIONS:/note= "terminateur du phage fd"
   (ix) CARACTERISTIQUE:
      (A) NOM/CLE: misc RNA
      (B) EMPLACEMENT:1254..2505
      (D) AUTRES INFORMATIONS:/note= "gene codant pour le répresseur de l'operon lactose"
   (ix) CARACTERISTIQUE:
      (A) NOM/CLE: misc_RNA
      (B) EMPLACEMENT:2506..4410
      (D) AUTRES INFORMATIONS:/note= "séquence de pBR 327"
   (xi) DESCRIPTION DE LA SEQUENCE: SEQ ID NO: 1:
   TCGAGTGGGT TTGAGGCGAT CACACTTCTG TTAACGCAGA ACCTAAACGC ATCTCGACTG    60
   CACGGTGCAC CAATGCTTCT GGCGTCAGGC AGCCATCGGA AGCTGTGGTA TGGCTGTGCA   120
   GGTCGTAAAT CACTGCATAA TTCGTGTCGC TCAAGGCGCA CTCCCGTTCT GGATAATGTT   180
   TTTTGCGCCG ACATCATAAC GGTTCTGGCA AATATTCTGA AATGAGCTGT TTCGAGCTGA   240
   CTGACTGTTG CTTATATTAC ATCGATAGCG TATAATGTGT GGAATTGTGA GCGGATAACA   300
   ATTTCACACA GTTTTTCGCG AAGAAGGAGA TATACATATG AAAAAGATCC TGGCGTTAGC   360
   TGCGCTGACT ACCGTTGTAT TCTCTGCGTC CGCCTTCGCT GGCCCTGTGC CTCCCAGTAC   420
   TGCCCTCAGG GAGCTCATTG AGGAGCTGGT CAACATCACC CAGAACCAGA AGGCTCCGCT   480
   CTGCAATGGC AGCATGGTAT GGAGCATCAA CCTGACAGCT GGCATGTACT GTGCAGCCCT   540
   GGAATCCCTG ATCAACGTGT CAGGCTGCAG TGCCATCGAG AAGACCCAGA GGATGCTGAG   600
   CGGATTCTGC CCGCACAAGG TCTCAGCTGG GCAGTTTTCC AGCTTGCATG TCCGAGACAC   660
   CAAAATCGAG GTGGCCCAGT TTGTAAAGGA CCTGCTCTTA CATTTAAAGA AACTTTTTCG   720
   CGAGGGACGG TTCAACTGAA ACTTCGAAAG CATCATTATT TGGGATCCGG CTGCTAACAA   780
   AGCCCGAAAG GAAGCTGAGT TGGCTGCTGC CACCGCTGAG CAATAACTAG CATAACCCCT 840
   TGGGGCCTCT AAACGGGTCT TGAGGGGTTT TTTGCTGAAA GGAGGAACTA TATCCGGATG 900
   TACCAAGCTT GGCCGGATCA AAGTTTTGTC GTCTTTCCAG ACGTTAGTAA ATGAATTTTC 960
   TGTATGAGGT TTTGCTAAAC AACTTTCAAC AGTTTCAGCG GAGTGAGAAT AGAAAGGAAC 1020
   AACTAAAGGA ATTGCGAATA ATAATTTTTT CACGTTGAAA ATCTCCAAAA AAAAAGGCTC 1080
   CAAAAGGAGC CTTTAATTGT ATCGGTTTAT CAGCTTGCTT TCGAGGTGAA TTTCTTAAAC 1140
   AGCTTGATAC CGATAGTTGC GCCGACAATG ACAACAACCA TCGCCCACGC ATAACCGATA 1200
   TATTCGGTCG CTGAGGCTTG CAGGGAGTCA AAGGCCGCTT TTGCGGGATC GATCCGCGGA 1260
   AGCATAAAGT GTAAAGCCTG GGGTGCCTAA TGAGTGAGCT AACTCACATT AATTGCGTTG 1320
   CGCTCACTGC CCGCTTTCCA GTCGGGAAAC CTGTCGTGCC AGCTGCATTA ATGAATCGGC 1380
   CAACGCGCGG GGAGAGGCGG TTTGCGTATT GGGCGCCAGG GTGGTTTTTC TTTTCACCAG 1440
   TGAGACGGGC AACAGCTGAT TGCCCTTCAC CGCCTGGCCC TGAGAGAGTT GCAGCAAGCG 1500
   GTCCACGCTG GTTTGCCCCA GCAGGCGAAA ATCCTGTTTG CTGGTGGTTA ACGGCGGGAT 1560
   ATAACATGAG CTGTCTTCGG TATCGTCGTA TCCCACTACC GAGATATCCG CACCAACGCG 1620
   CAGCCCGGAC TCGGTAATGG CGCGCATTGC GCCCAGCGCC ATCTGATCGT TGGCAACCAG 1680
   CATCGCAGTG GGAACGATGC CCTCATTCAG CATTTGCATG GTTTGTTGAA AACCGGACAT 1740
   GGCACTCCAG TCGCCTTCCC GTTCCGCTAT CGGCTGAATT TGATTGCGAG TGAGATATTT 1800
   ATGCCAGCCA GCCAGACGCA GACGCGCCGA GACAGAACTT AATGGGCCCG CTAACAGCGC 1860
   GATTTGCTGG TGACCCAATG CGACCAGATG CTCCACGCCC AGTCGCGTAC CGTCTTCATG 1920
   GGAGAAAATA ATACTGTTGA TGGGTGTCTG GTCAGAGACA TCAAGAAATA ACGCCGGAAC 1980
   ATTAGTGCAG GCAGCTTCCA CAGCAATGGC ATCCTGGTCA TCCAGCGGAT AGTTAATGAT 2040
   CAGCCCACTG ACGCGTTGCG CGAGAAGATT GTGCACCGCC GCTTTACAGG CTTCGACGCC 2100
   GCTTCGTTCT ACCATCGACA CCACCACGCT GGCACCCAGT TGATCGGCGC GAGATTTAAT 2160
   CGCCGCGACA ATTTGCGACG GCGCGTGCAG GGCCAGACTG GAGGTGGCAA CGCCAATCAG 2220
   CAACGACTGT TTGCCCGCCA GTTGTTGTGC CACGCGGTTG GGAATGTAAT TCAGCTCCGC 2280
   CATCGCCGCT TCCACTTTTT CCCGCGTTTT CGCAGAAACG TGGCTGGCCT GGTTCACCAC 2340
   GCGGGAAACG GTCTGATAAG AGACACCGGC ATACTCTGCG ACATCGTATA ACGTTACTGG 2400
   TTTCACATTC ACCACCCTGA ATTGACTCTC TTCCGGGCGC TATCATGCCA TACCGCGAAA 2460
   GGTTTTGCGC CATTCGATCT ACGCCGGACG CATCGTGGCC GCAAACCAAC CCTTGGCAGA 2520
   ACATATCCAT CGCGTCCGCC ATCTCCAGCA GCCGCACGCG GCGCATCTCG GGCCGCGTTG 2580
   CTGGCGTTTT TCCATAGGCT CCGCCCCCCT GACGAGCATC ACAAAAATCG ACGCTCAAGT 2640
   CAGAGGTGGC GAAACCCGAC AGGACTATAA AGATACCAGG CGTTTCCCCC TGGAAGCTCC 2700
   CTCGTGCGCT CTCCTGTTCC GACCCTGCCG CTTACCGGAT ACCTGTCCGC CTTTCTCCCT 2760
   TCGGGAAGCG TGGCGCTTTC TCAATGCTCA CGCTGTAGGT ATCTCAGTTC GGTGTAGGTC 2820
   GTTCGCTCCA AGCTGGGCTG TGTGCACGAA CCCCCCGTTC AGCCCGACCG CTGCGCCTTA 2880
   TCCGGTAACT ATCGTCTTGA GTCCAACCCG GTAAGACACG ACTTATCGCC ACTGGCAGCA 2940
   GCCACTGGTA ACAGGATTAG CAGAGCGAGG TATGTAGGCG GTGCTACAGA GTTCTTGAAG 3000
   TGGTGGCCTA ACTACGGCTA CACTAGAAGG ACAGTATTTG GTATCTGCGC TCTGCTGAAG 3060
   CCAGTTACCT TCGGAAAAAG AGTTGGTAGC TCTTGATCCG GCAAACAAAC CACCGCTGGT 3120
   AGCGGTGGTT TTTTTGTTTG CAAGCAGCAG ATTACGCGCA GAAAAAAAGG ATCTCAAGAA 3180
   GATCCTTTGA TCTTTTCTAC GGGGTCTGAC GCTCAGTGGA ACGAAAACTC ACGTTAAGGG 3240
   ATTTTGGTCA TGAGATTATC AAAAAGGATC TTCACCTAGA TCCTTTTAAA TTAAAAATGA 3300
   AGTTTTAAAT CAATCTAAAG TATATATGAG TAAACTTGGT CTGACAGTTA CCAATGCTTA 3360
   ATCAGTGAGG CACCTATCTC AGCGATCTGT CTATTTCGTT CATCCATAGT TGCCTGACTC 3420
   CCCGTCGTGT AGATAACTAC GATACGGGAG GGCTTACCAT CTGGCCCCAG TGCTGCAATG 3480
   ATACCGCGAG ACCCACGCTC ACCGGCTCCA GATTTATCAG CAATAAACCA GCCAGCCGGA 3540
   AGGGCCGAGC GCAGAAGTGG TCCTGCAACT TTATCCGCCT CCATCCAGTC TATTAATTGT   3600
   TGCCGGGAAG CTAGAGTAAG TAGTTCGCCA GTTAATAGTT TGCGCAACGT TGTTGCCATT   3660
   GCTGCAGGCA TCGTGGTGTC ACGCTCGTCG TTTGGTATGG CTTCATTCAG CTCCGGTTCC   3720
   CAACGATCAA GGCGAGTTAC ATGATCCCCC ATGTTGTGCA AAAAAGCGGT TAGCTCCTTC   3780
   GGTCCTCCGA TCGTTGTCAG AAGTAAGTTG GCCGCAGTGT TATCACTCAT GGTTATGGCA   3840
   GCACTGCATA ATTCTCTTAC TGTCATGCCA TCCGTAAGAT GCTTTTCTGT GACTGGTGAG   3900
   TACTCAACCA AGTCATTCTG AGAATAGTGT ATGCGGCGAC CGAGTTGCTC TTGCCCGGCG   3960
   TCAACACGGG ATAATACCGC GCCACATAGC AGAACTTTAA AAGTGCTCAT CATTGGAAAA   4020
   CGTTCTTCGG GGCGAAAACT CTCAAGGATC TTACCGCTGT TGAGATCCAG TTCGATGTAA   4080
   CCCACTCGTG CACCCAACTG ATCTTCAGCA TCTTTTACTT TCACCAGCGT TTCTGGGTGA   4140
   GCAAAAACAG GAAGGCAAAA TGCCGCAAAA AAGGGAATAA GGGCGACACG GAAATGTTGA   4200
   ATACTCATAC TCTTCCTTTT TCAATATTAT TGAAGCATTT ATCAGGGTTA TTGTCTCATG   4260
   AGCGGATACA TATTTGAATG TATTTAGAAA AATAAACAAA TAGGGGTTCC GCGCACATTT   4320
   CCCCGAAAAG TGCCACCTGA CGTCTAAGAA ACCATTATTA TCATGACATT AACCTATAAA   4380
   AATAGGCGTA TCACGAGGCC CTTTCGTCCC                                    4410